Europäisches Patentamt

European Patent Office

Office européen des brevets

(11) Publication number: **0 413 056 A1**

# EUROPEAN PATENT APPLICATION

(21) Application number: 89115299.3

(51) Int. Cl.5: **A23L 1/211, A61K 47/12**

(22) Date of filing: 18.08.89

(43) Date of publication of application:
20.02.91 Bulletin 91/08

(84) Designated Contracting States:
AT BE CH DE ES FR GB GR IT LI LU NL SE

(71) Applicant: NIPPON HYPOX LABORATORIES
INCORPORATED
1759, Matsugaya Hachioji-shi
Tokyo(JP)

(72) Inventor: Satoh, Toshio
57-3, Nagao, Jyoroku-cho
Tokushima-shi Tokushima-ken(JP)
Inventor: Matsumoto, Hitoshi
125-22, Shimofukuman Hachiman-cho
Tokushima-shi Tokushima-ken(JP)
Inventor: Kakegawa, Hisao
No. 307, City-Corpo. Kimura 3-59,
Okihama-Higashi
Tokushima-shi Tokushima-ken(JP)
Inventor: Niiro, Yasunori No. 403, 2nd
Masuoka Bldg.
1-3, Minamisako-hachiban-cho
Tokushima-shi Tokushima-ken(JP)

(74) Representative: Hansen, Bernd, Dr.rer.nat. et
al
Hoffmann, Eitle & Partner Patentanwälte
Arabellastrasse 4 Postfach 81 04 20
D-8000 München 81(DE)

(54) Method for reducing astringency and bitterness of tannic acid.

(57) The present invention relates to a method for reducing astringency and bitterness of tannic acid, which comprises adding an effective amount of an organic acid and/or a salt thereof to tannic acid.

EP 0 413 056 A1

# METHOD FOR REDUCING ASTRINGENCY AND BITTERNESS OF TANNIC ACID

## BACKGROUND OF THE INVENTION

### (1) Field of the Invention

The present invention relates to a method for reducing astringency and bitterness (astringent and bitter tastes) of tannic acid.

### (2) Background Art

Tannic acid is a substance extracted from a Japanese gall or nutgall and has been used for long time as astringent or hemostatic. It has been also reported that tannic acid has the anti-allergic and anti-viral actions.

Thus, tannic acid is useful as a medicinal substance but has a very strong astringency and bitterness peculiar to this acid, so that when it is put into the mouth, its astringency and bitterness would be diffused in the mouth to give an unpleasant feeling. Medicamental application of tannic acid in the mouth was therefore quite objectionable.

## SUMMARY OF THE INVENTION

Accordingly, the object of this invention is to provide a method for reducing astringency and bitterness of tannic acid so as to enable medicamental application of tannic acid in the mouth without causing any unpleasant feeling.

As a result of extensive researches for a substance which serves for reducing astringency and bitterness of tannic acid when mixed therewith, the present inventors found that astringency and bitterness of tannic acid can be reduced by adding an effective amount of an organic acid and/or a salt thereof to tannic acid, and that the sourness of the organic acid itself is also abated when such organic acid is mixed with tannic acid. This finding has enabled attainment of the object of this invention.

Thus, the present invention provides a method for reducing a stringency and bitterness of tannic acid, which comprises adding an effective amount of an organic acid and/or a salt thereof to tannic acid.

The invention will be described in detail below.

The tannic acid to be treated in this invention is preferably the one obtained from extraction of a Japanese gall or nutgall.

The organic acid and/or salt thereof used for reducing astringency and bitterness of tannic acid in this invention is preferably selected from citric acid, tartaric acid, succinic acid, gluconic acid, glucuronic acid, ascorbic acid, isoascorbic acid, glutamic acid, aspartic acid, malic acid and metal salts (e.g. sodium salt, potassium salt, ammonium salt, etc.) of these acids. The amount of such organic acid and/or a salt thereof to be used, although variable depending on the type of the organic acid used, is usually more than twice the amount of tannic acid by weight for effecting the reduction of astringency and bitterness of tannic acid as well as the abating of sourness of the organic acid itself. Most preferably, said organic acid and/or its salt is used in an amount more than 5 times the amount of tannic acid by weight.

The tannic acid which has been reduced in its astringency and bitterness by the addition of an organic acid and/or a salt thereof may be further added with the pharmaceutically acceptable additives such as carrier, excipient, builder or filler, savoring agent, binder, lubricant, etc., and can be worked into pharmaceutical preparations in the form of tablets, powders, granules, syrups, medicaments for oral application, etc.

The tannic acid treated acoording to this invention is preferably used for oral application in the form of troches, salves or ointments, pastes, etc. Use in the form of troches, especially the one weighing about 0.5 to 2 g, is most preferred.

The present invention will hereinafter be described in further detail by showing the examples thereof.

Example 1

Various savoring substances were added to tannic acid to examine whether astringency and bitterness of tannic acid are reduced by the addition of such substances.

Used as the savoring substances were tartaric acid (as an organic acid), berberine chloride, chilly pepper and sucrose. Tartaric acid and sucrose were added in an amount of 10 parts by weight and berberine chloride and chilly pepper in an amount of one part by weight to one part by weight of tannic acid.

A palate test was conducted with 12 subjects on each of the blends of tannic acid and said respective savoring substances for determining whether astringency and bitterness of tannic acid were reduced by the addition of said substances. In the palate test, the amount of tannic acid taken by the subjects was the same (about 1 mg) in all the cases. The same applies to the succeeding Examples).

The results are shown in Table 1.

Table 1

| Number of subjects: 12 | | |
|---|---|---|
| | Astringency | Bitterness |
| Tannic acid and berberine chloride | + + (6/12), --(6/12) | + + + (6/12), + + (6/12) |
| Tannic acid and chilly pepper | + + (6/12), -(6/12) | ±(6/12), -(6/12) |
| Tannic acid and sucrose | + + + (9/12), + + (3/12) | + + + (6/12), + + (6/12) |
| Tannic acid and tartaric acid | ±(3/12), -(9/12) | + (3/12), ±(3/12), -(6/12) |
| (Note) Grade: | | |
| + + + very strong<br>+ + strong<br>+ sensible<br>± almost insensible<br>- totally insensible | | |

As seen from Table 1, the addition of tartaric acid to tannic acid produced a prominent astringency and bitterness reducing effect. Although the blend of tannic acid and tartaric acid had a sourness originating from tartaric acid, such sourness was milder than that of tartaric acid itself, and thus the blend was suited for the preparation of medicaments for oral application (especially in the from of troches).

On the other hand, the blend of tannic acid and chilly pepper, although reduced in astringency and bitterness of tannic acid, had a pungent taste and was unsuited for use as a medicament for oral application.

The addition of berberine chloride to tannic acid could reduce astringency of tannic acid to some extent but produced no bitterness reducing effect.

The above results show that the greatest effect for reducing astringency and bitterness of tannic acid is obtained when tartaric acid, which is an organic acid, is added to tannic acid.

Example 2

By using ascorbic acid and citric acid as the test organic acids, their effect of reducing astringency (bitterness) of tannic acid was examined by varying the amount of said organic acids added to tannic acid. 21 subjects took part in the test.

The results are shown in Tables 2(1) and 2(2)

Table 2(1)

| Number of subjects: 21 | | |
|---|---|---|
| Blend | Sourness | Astringency (bitterness) |
| Tannic acid : ascorbic acid | | |
| 1 : 0.1 | -(21/21) | + + +(12/21), + +(9/21) |
| 1 : 1 | +(9/21), -(12/21) | + + +(18/21), + +(3/21) |
| 1 : 3 | +(6/21), ±(15/21) | + + +(18/21), + +(3/21) |
| 1 : 10 | +(9/21), ±(12/21) | +(15/21), ±(3/21), -(3/21) |
| 1 : 30 | + +(12/21), +(9/21) | +(6/21), ±(9/21), -(6/21) |
| 1 : 100 | + + +(6/21), + +(15/21) | ±(9/21), -(12/21) |

Table 2(2)

| Number of subjects: 21 | | |
|---|---|---|
| Blend | Sourness | Astringency (bitterness) |
| Tannic acid : ascorbic acid | | |
| 1 : 0.1 | -(21/21) | + + +(9/21), + +(12/21) |
| 1 : 1 | +(9/21), ±(6/21) -(6/21) | + +(18/21), +(3/21) |
| 1 : 2 | +(21/21) | + +(12/21), +(9/21) |
| 1 : 5 | + +(6/21), +(15/21) | + +(3/21), +(18/21) |
| 1 : 10 | + + +(12/21), + +(6/21), +(3/21) | ±(3/21), -(18/21) |
| (Note) The grades (+ + +, + +, +, ± and -) are the same as described in Table 1. The numerals in the parentheses show the number of the subjects who gave the shown grades and the total number of the subjects participating in the test. | | |

From Tables 2(1) and 2(2), the effect of ascorbic acid and citric acid for reducing astringency (bitterness) of tannic acid is evident. In the case of asoorbic acid, a particularly remarkable effect was noted when it was used in an amount exceeding 5 times, especially in an amount of 10 times or more the amount of tannic acid by weight, and in the case of citric acid, a prominent effect was obtained when it was used in an amount of two times or more, especially 5 times or more the amount of tannic acid by weight.

Example 3

A 24 subjects palate test was conducted on the blends of tannic acid with 10 times by weight as much amount of citric acid, its sodium salt, malic acid, tartaric acid and ascorbic acid.
The results are shown in Table 3.

Table 3

| Number of subjects: 24 | | |
|---|---|---|
| Blend | Sourness | Astringency (bitterness) |
| Tannic acid : sodium citrate (1 : 10)<br>Tannic acid : citric acid (1 : 10)<br>Tannic acid : malic acid (1 : 10)<br>Tannic acid : tartaric acid (1 : 10)<br>Tannic acid : ascorbic acid (1 : 10) | + (6/24), ±(6/24), -(12/24)<br>+ + +(12/24), + +(12/24)<br>+ + +(12/24), + +(12/24)<br>+ + +(12/24), + +(12/24)<br>+ +(18/24), +(6/24) | + (3/24), ±(6/24), -(15/24)<br>+ (3/24), -(21/24)<br>+ (3/24), ±(9/24), -(12/24)<br>+ (3/24), ±(6/24), -(15/24)<br>+ +(3/24), +(3/24), ±(9/24), -(9/24) |
| (Note) The grades (+ + +, + +, +, ± and -) are the same as described in Table 1. The numerals in the parentheses show the number of the subjects who gave the shown grades and the total number of the subjects participating in the test. | | |

The results of Table 3 attest to the prominent effect of reducing astringency (bitterness) of tannic acid by said organic acids, viz. citric acid, malic acid, tartaric acid and ascorbic acid. It is noteworthy that sodium citrate, an organic acid salt, also has an effect of reducing astringency (bitterness) of tannic acid. This is significant in view of the following fact: sodium citrate is weak in sourness in comparison with citric acid, and the fact that such low-sourness sodium citrate can reduce astringency (bitterness) of tannic acid implies that the tannic acid astringency (bitterness) reducing effect does not merely depend on masking of astringency (bitterness) of tannic acid by sourness of the organic acid.

Shown below are the examples of recipes for the preparation of troches by using tannic acid which has been reduced in astringency and bitterness by the method of this invention.

| Formulation Example 1 | |
|---|---|
| | Content per tablet |
| Tannic acid | 50 mg |
| Ascorbic acid (organic acid) | 750 mg |
| Lactose (excipient) | 877.5mg |
| Starch | 225 mg |
| Talc | 15 mg |
| Magnesium stearate | 7.5mg |

| Formulation Example 2 | |
|---|---|
| | Content per tablet |
| Tannic acid | 50 mg |
| Citric acid (organic carboxylic acid) | 375 mg |
| White sugar (excipient) | 150 mg |
| Lactose (excipient) | 997.5mg |
| Starch | 225 mg |
| Talc | 15 mg |
| Magnesium stearate | 7.5mg |

The troches prepared according to said formulation examples, when licked in the mouth, had no astringency and bitterness of tannic acid, and also in these troches the sourness of the organic acid has been moderately weakened. It was thus confirmed that these troches had no problem in their oral application.

As described above, in accordance with this invention, it is possible to markedly reduced astringency and bitterness of tannic acid by adding an effective amount of a specific organic acid and/or a salt thereof to tannic acid having peculiar astringent and bitter tastes.

**Claims**

1. A method for reducing astringency and bitterness of tannic acid, which comprises adding an effective amount of an organic acid and/or a salt thereof to tannic acid.

2. A method according to Claim 1, wherein said tannic acid is the one obtained from extraction of a Japanese gall or nutgall.

3. A method according to Claim 1, wherein said organic acid is at least one selected from the group consisting of citric acid, tartaric acid, succinic acid, gluconic acid, glucuronic acid, ascorbic acid, isoascorbic acid, glutamic acid, aspartic acid and malic acid.

4. A method according to Claim 1, wherein said salt of organic acid is at least one selected from the group consisting of sodium salt, potassium salt and ammonium salt.

5. A method according to Claim 1, wherein the amount of said organic acid and/or said salt thereof is more than twice the amount of tannic acid by weight.

6. A method according to Claim 1, wherein the amount of said organic acid and/or said salt thereof is more than 5 times the amount of tannic acid by weight.

7. A formulation obtained by adding to tannic acid, an organic acid and/or a salt thereof in such an amount that astringency and bitterness of tannic acid can be reduced.

8. A formulation according to Claim 7, which is used for the preparation of medicaments for oral application.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | DE-A-1492744 (KOCH K.H.)<br>* the whole document *<br>--- | 1-8 | A23L1/211<br>A61K47/12 |
| Y | FR-A-2269352 (AB DRACO)<br>* the whole document *<br>--- | 1-8 | |
| Y | US-A-3660111 (KOCH K. H.)<br>* the whole document *<br>----- | 1-8 | |

TECHNICAL FIELDS
SEARCHED (Int. Cl.5)

A61K
A23F
A23L

The present search report has been drawn up for all claims

| Place of search | Date of completion of the search | Examiner |
|---|---|---|
| BERLIN | 06 MARCH 1990 | AVEDEKIAN P. F. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................................................
& : member of the same patent family, corresponding
    document

EPO FORM 1503 03.82 (P0401)